# EUROPEAN PATENT APPLICATION

(11) **EP 1 728 523 A1**
(43) Date of publication of application: **06.12.2006**
(21) Application number: 06380153.4
(22) Date of filing: 02.06.2006
(51) Int. Cl.: A61L 9/03

(54) **Multiuse air freshener**

(30) Priority: 03.06.2005 ES 200501274 U
(71) Applicant: Igual Alonso, Jose Luis, 03007 Alicante (ES)
(72) Inventor: Igual Alonso, Jose Luis, 03007 Alicante (ES)
(74) Representative: Munoz Garcia, Antonio

(57) **Abstract**

The present invention relates to a multiuse air freshener, the casing of which has a vertical, planar rear wall suitable for detachably or irremovably fixing indistinctly to a plug socket or wall, said wall having an extension for the rear area of the container of the product, having a planar horizontal base; wherein the lower extension of the wall has orifices for the irremovable wall-mounting by means of screws; wherein there is arranged in said wall an orifice for a jack selectively usable and connectable to the power supply circuit of the heating resistance; wherein there are arranged in the upper area of said wall "keyhole" orifices which are complementary to the respective lugs with an expanded free end and integral with a small planar support which can be fixed with screws, and wherein when the casing is wall-mounted, the resistance circuit ends in a cable with a jack plug.

## Description

### OBJECT OF THE LA INVENTION

The present invention relates to an air freshener of the type able to supply to the surrounding area discharges of a deodorizing, aromatic, humidifying product etc., structured so as to provide a wide range of practical use possibilities, and more specifically so as to be usable as a desk-top element, as an irremovable wall-mounted element, as a detachable wall-mounted element, and as an element fixable to a conventional plug socket.

### BACKGROUND TO THE INVENTION

Air fresheners are known which have a small bottle or container of the air-freshening product in question, projecting from which there is a wick passing through a heating element with an electrical resistance such that the heat generated by said heating elements enhances the evaporation of the product absorbed by the wick, which exits outwardly through the corresponding grid.

There are air fresheners based on this basic structure that are intended to be fixed to a conventional wall plug socket, for which purpose they have a plug on their rear wall; air fresheners are also known the casing of which has orifices allowing their screwing to anchors previously inserted in the wall, in which case the plug is not built into the casing of the apparatus but ending in an electric cable of undetermined length, and air fresheners are also known having a planar base, allowing the arrangement thereof on the table or any other horizontal surface or the like.

However, no air freshener is known which is multiuse, that is, one that can be used in any of the ways hereinbefore described.

### DESCRIPTION OF THE INVENTION

The air freshener object of the present invention fills such gap, having a multiuse character which, as has been mentioned previously, allows the arrangement thereof as a desk-top element on a planar surface, as an irremovable wall-mounted element, as a detachable wall-mounted element also, and finally as an element that can be fixed to a conventional plug socket through its own plug.

To that end and more specifically, said air freshener incorporates a casing in which there is defined a rear planar wall provided with a lower extension, and a lower base open for the most part to which the container of the product is coupled, which has the particular feature of in turn incorporating a planar support base which is of considerable width, which is flush to the surface or exceeds the lower extension of the rear wall and which accordingly defines a wide support surface with optimal features for the use of the air freshener as a desk-top element.

Said vertical, planar rear wall has on its lower extension a pair of orifices allowing the screwing of the casing and accordingly the entire air freshener, to a vertical face, such as a wall, in which the complementary anchors have already been inserted.

In said vertical, planar rear wall there is also arranged a wide circular orifice allowing the optional coupling of a jack plug, which in the case of being used, that is, in the case of being operative as such a connection element, it must in turn be duly connected to the inner power supply circuit of the resistance.

Finally, and according to another feature of the invention, in the upper area of the vertical, planar rear wall of the casing there is arranged a pair of "keyhole" orifices, allowing the coupling of a pair of lugs with an expanded free end, projecting from a substantially planar ancillary support which can be fixed to the wall by screwing, such that it allows the assembly/disassembly thereon of the casing of the air freshener.

### DESCRIPTION OF THE DRAWINGS

As a complement to the description being made and for the purpose of aiding to better understand the features of the invention according to a preferred practical embodiment thereof, a set of drawings is attached as an integral part of said description which, with an illustrative and nonlimiting character, represent the following:
Figure 1 shows a front side perspective view of a multiuse air freshener carried out according to the object of the present invention.

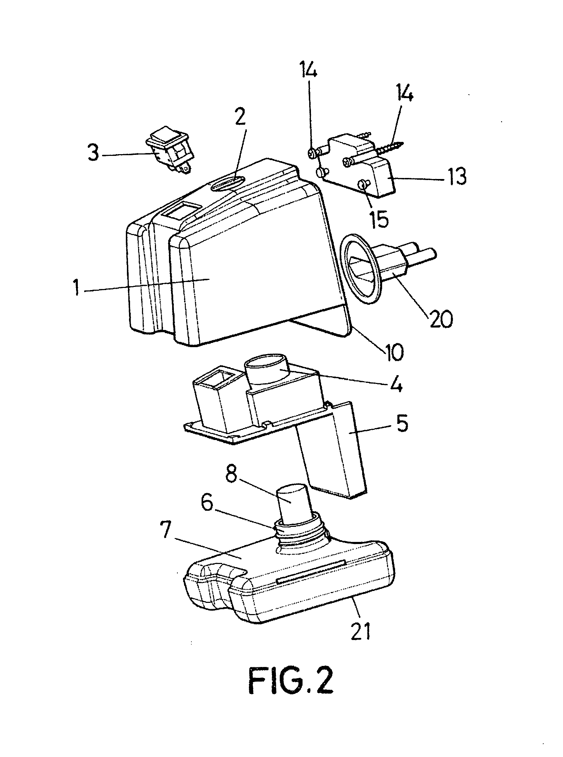

Figure 2 shows an exploded, also perspective view of the assembly represented in the previous figure.

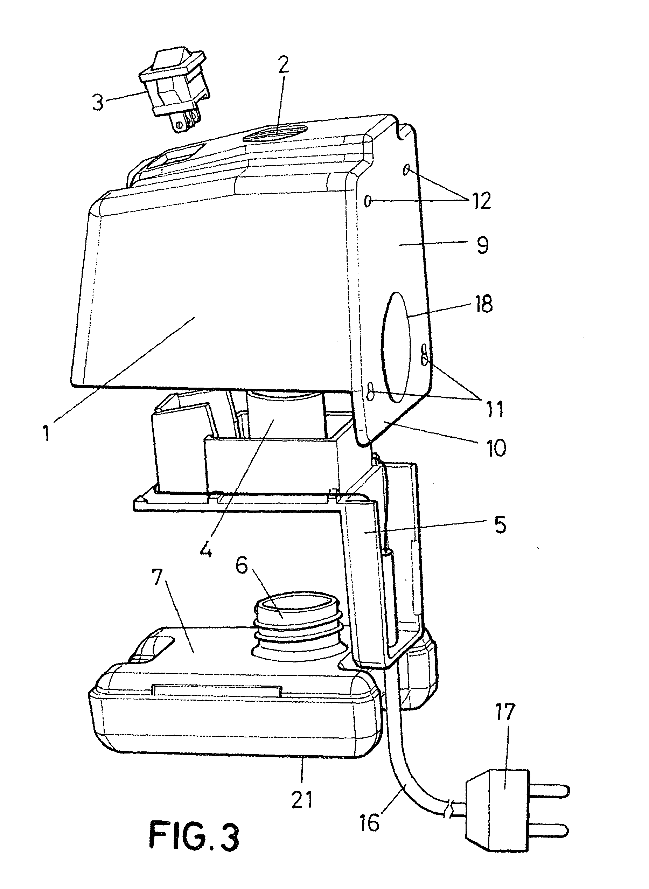

Figure 3 shows anther exploded perspective view of the assembly, turned with respect to the one of the previous figure so as to show the rear area of the air freshener.

### PREFERRED EMBODIMENT OF THE INVENTION

In view of the figures discussed, it can be observed how the air freshener proposed by the invention is constituted, like any conventional air freshener of this type, from a casing (1) with an upper grid (2) for the outlet of the air freshening product discharge, and with a switch (3) for closing the electrical power supply circuit of a heating resistance, surrounding a neck (4) belonging to an inner support (5), that can be fixed in the casing (1) and opposite to the neck (6) of the container or bottle (7) supplying the product, projecting from which there is a thick wick (8) which, located inside the neck (4), is affected by the heat dissipated by the electric resistor or resistors.

Based on this conventional basic structure and according to the invention, the air freshener focuses its features on the fact that it incorporates in its casing a planar rear wall (9), which is vertical in the air freshener operating position, provided with a lower extension (10) in which there is arranged a pair of orifices (11) allowing the wall-mounting of the casing (1) and accordingly of the air freshener as a whole, with the aid of respective long bolts threaded in anchors previously inserted in the wall.

There are arranged in the upper area of said rear wall (9) of the casing two other orifices (12), in this case "keyhole" orifices, these orifices being intended to allow the detachable wall-mounting of the air freshener, specifically with the aid of a small planar support (13), which is irremovably mounted to the wall with the aid of long bolts (14), and having on its front face a pair of lugs (15) with an expanded free end, able to enter the lower area of said orifices (12) of the casing and blocking the latter when they move up towards the upper area of such orifices.

In both cases, that is, both when the casing is irremovably wall-mounted through the openings (11) and when it is detachably mounted to the support or wall (13), the power supply circuit of the heating resistances surrounding the neck (4) passes through the grooved vertical branch of the inner support (5) and projects outwardly in the form of a cable (16) of a suitable length, ending in the corresponding jack plug (17).

Nevertheless, it has been provided that the air freshener can also be fixed to a conventional wall plug socket, for which purpose it has a wide orifice (18) on its rear wall (9), specifically at the lower level, in which there can be coupled a jack plug (20) that is integral with the casing (1) and connected to said circuit of the heating resistances, such that the integral fixing of this jack plug (20) allows the connection thereof to a plug socket while at the same time it acts as a mechanical support for the assembly.

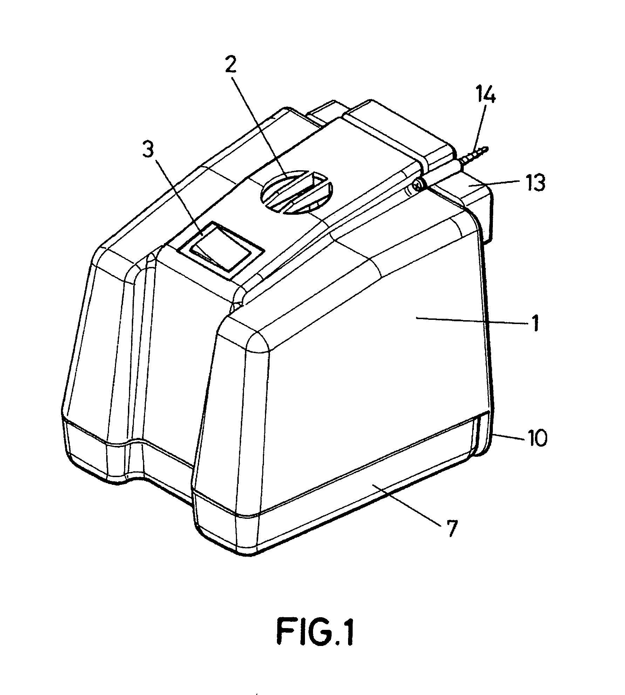

Finally and as particularly observed in Figure 1, said container (7) has a wide lower base (21) located on the plane corresponding to the lower edge of the extension (10) of the rear face (9) of the casing (1), such that said base (21) of the container (7) is usable as a support base for the air freshener as a whole when it is to be used as a table-top element, and evidently in this case, it must have said cable (16) ending in its plug (17) for the connection to the general electric supply network of the home or room in which it is located.

Therefore and as repeatedly stated, a single air freshener can be a table-top element to be fixed directly to the plug socket providing the electric power, or it can be either detachably or irremovably wall-mounted.

## Claims

1. A multiuse air freshener of the type incorporating a casing inside of which there is arranged a support for the mechanisms of the air freshener, in particular for a heating resistance and its power supply circuit, in which there is arranged a neck for a wick which absorbs the air freshening product from a container which can be coupled to said casing and in which the heating resistance is connected to the general electric supply network, **characterized in that** the casing thereof has a vertical, planar rear wall provided with means for indistinctly fixing said casing to a plug socket or to a wall, in the latter case detachably or irremovably, said rear wall having a lower extension to which adapts the rear part of the container of the product, which in turn has a wide planar horizontal base, assuring the stability of the air freshener as a desk-top element.

2. A multiuse air freshener according to claim 1, **characterized in that** the lower extension of the rear wall of the casing is provided with a pair of orifices for the irremovable wall-mounting of the air freshener by means of screws or long bolts.

3. A multiuse air freshener according to the previous claims, **characterized in that** in the lower area of the rear wall of the casing, there is arranged a wide circular orifice which may be mechanically coupled with a jack plug which is selectively useable and, as the case may be, connectable to the power supply circuit of the heating resistance.

4. A multiuse air freshener according to the previous claims, **characterized in that** in the in the upper part of the rear wall of the casing, there are arranged a pair of "keyhole" orifices complementary to which are the respective lugs with an expanded free end and integral with a small planar support, which may be wall-mounted with the aid of screws or long bolts.

5. A multiuse air freshener according to the previous claims, **characterized in that** when the casing is detachably or irremovably wall-mounted, the power supply circuit of the heating resistance ends in a wire which extends substantially outside the casing of the air freshener and which, with an undefined length, ends in a jack plug.
